# EUROPEAN PATENT APPLICATION

(11) **EP 4 389 879 A1**
(43) Date of publication of application: **26.06.2024**
(21) Application number: 22215536.8
(22) Date of filing: 21.12.2022
(51) Int. Cl.: C12N 7/00, B01D 63/08, B01D 71/10

(54) **PURIFICATION OF VIRUSES, VIRUS-LIKE PARTICLES, AND GLOBULAR BIOMOLECULES BY FILTRATION-SUPPORTED POLYALKYLENE GLYCOL PRECIPITATION**

(71) Applicant: Sartorius Stedim Biotech GmbH, 37079 Göttingen (DE)
(72) Inventor: PFLANZ, Karl, 37079 Göttingen (DE); LABISCH, Jennifer, 37079 Göttingen (DE)
(74) Representative: Müller-Boré & Partner Patentanwälte PartG mbB

(57) **Abstract**

The present invention relates to methods for the purification of viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, by filtrationsupported polyalkylene glycol precipitation, and to filtration modules that can be used in said methods.

## Description

The present invention relates to methods for the purification of viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, by filtration-supported polyalkylene glycol precipitation, and to filtration modules that can be used in said methods.

Viral vectors play an increasing role as vectors in gene and/or cell therapy. For example, lentiviral vectors (LV) are one of the three most widely represented viral vector systems in clinical trials of gene therapy and gene-modified cell therapy. Their relatively low stability challenges the downstream process. Thus, constant development and improvement of gentle process steps are required. Steric exclusion chromatography (SXC) has been gaining interest in the field of viral vector purification, but scale-up has not yet been addressed, raising the question of how this can be accomplished.

LV have long been used in the biopharmaceutical industry, primarily in gene-modified cell therapy. Stable integration of the LV genome and long-term expression of the transgene achieve a successful therapeutic outcome for certain diseases like acute lymphoblastic leukemia (ALL). The first pediatric patient with ALL treated with LV-based gene-modified cell therapy is now ten years cancer free. In clinical trials, LV are used to treat a wide range of diseases, including cancers, immune disorders, metabolic disorders, and rare congenital diseases. New potential applications for LV have emerged. Recently, the use of LV gained importance as a possible vaccination platform using integrating as well as non-integrating LV to target infectious diseases. The broad range of diseases that can be treated with LV and emerging applications will lead to an increased need for efficient LV bioprocessing. Many challenges are faced during LV manufacturing, especially purification requires further optimization.

The use of SXC for LV purification was recently published. A variety of viruses have been previously purified by SXC, including baculovirus, Orf virus, AAV, and influenza A virus. SXC is a gentle purification method offering high potential for the purification of large, enveloped, fragile viral vectors, as it does not require any chemical interaction between the target species and the stationary phase and preserves viral infectivity. The basic principle of SXC is known in the art. Briefly, the viral vector feed solution is mixed with polyethylene glycol (PEG) buffer and loaded onto a hydrophilic stationary phase. As such, SXC is a form of filtration-supported polyalkylene glycol precipitation. Upon the addition of PEG, depletion zones around the viral particles and the stationary phase are formed. These depletion zones are areas that are not accessible to the polymer's center of gravity. Hence, the PEG molecules are sterically excluded from these areas. The resulting depletion interaction results in the association of the viral vectors with the stationary phase. The viral particles are eluted with a PEG-free buffer, reversing the association of the viral particles with the stationary phase.

So far, SXC has only been performed on a small scale. SXC studies relying on membranes as a stationary phase used stacked membrane layers assembled in their housing (e.g., a stainless-steel holder for multi-use or an overmolded plastic housing for single-use) so that the flow is directed frontally from above, resulting in a dead-end flow. Membrane devices of a diameter between 13 mm and 25 mm with 10 to 20 layers of stacked membranes have been employed in previous publications of viral vector purification with SXC. However, a deep mechanistic understanding of the requirements of the membrane device, especially concerning the potential scale-up of SXC, is lacking. Current approaches use the typical chromatography model considering the total internal surface as interaction and binding area, whereby upscaling leads to an increase of flow path, an increase in pressure, and an increase process time. Both the location of viral vector association in the stationary phase and the effect of different membrane device geometries or sizes on the performance of SXC have yet to be investigated, leaving unanswered questions as to how a scale-up could be implemented.

Accordingly, the technical problem underlying the present invention is the provision of improved means for the purification of viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, using filtration-supported polyalkylene glycol precipitation, that are amenable to an up-scaling to intermediate to large production scales.

The solution to the above technical problem is achieved by the embodiments characterized in the claims.

In particular, in a first aspect, the present invention relates to a method for the purification of viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, by filtration-supported polyalkylene glycol precipitation, comprising the steps of:
(a) providing a filtration module, said filtration module comprising:
   (i-α) one stack or two separate stacks of 2 to 9 layers of a stabilized, non-ionic, hydrophilic porous structure, said layers being deposited on top of each other, wherein each of said stack(s) provide(s) a homogenous flow resistance and has a total thickness of 300 µm or more and 2000 µm or less, or
   (i-β) one monolithic stabilized, non-ionic, hydrophilic porous structure, or two separate monolithic stabilized, non-ionic, hydrophilic porous structures, wherein each of said porous structure(s) provide(s) a homogenous flow resistance and has a total thickness of 300 µm or more and 2000 µm or less, and
   (ii) a housing encasing said stack(s) of porous structure layers or said monolithic porous structure(s), said housing comprising a fluid inlet and a fluid outlet, wherein fluid entering the filtration module through the fluid inlet has to pass through said stack(s) of porous structure layers or said monolithic porous structure(s) in order to exit the filtration module through the fluid outlet, and wherein the housing is adapted to distribute the feed solution entering the housing via the fluid inlet evenly over the entire accessible surface area of the first layer of said stack(s) of porous structure layers or over the entire accessible surface area of said monolithic porous structure(s),
(b) providing a feed solution, containing said viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, to said filtration module via said fluid inlet at a surface area-dependent flow rate of at least 1.0 mL per minute per cm² of accessible surface area of the first layer of said stack(s) of layers of a porous structure or of the monolithic porous structure(s) (1.0 mL·min⁻¹·cm⁻²), whereby the viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, adhere to the porous structure, and
(c) eluting the viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, from said porous structure and thereby harvesting purified viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more.

According to step (a) of the methods of the present invention, a filtration module as defined above is provided, *inter alia* comprising (i) one stack or two separate stacks of 2 to 9, preferably 2 to 8, more preferably 3 to 7, more preferably 3 to 6, more preferably 3 to 5, layers of a stabilized, non-ionic, hydrophilic porous structure, or (ii) one or two separate monolithic stabilized, non-ionic, hydrophilic porous structure(s). As such, said filtration module can comprise 2, 3, 4, 5, 6, 7, 8 or 9 layers of a stabilized, non-ionic, hydrophilic porous structure, wherein 3, 4, or 5 layers of a stabilized, non-ionic, hydrophilic porous structure are particularly preferred. Said layers form a stack in which the layers are deposited on top of each other, preferably with maximum overlap. In this context, each stack of porous structure layers is (i) a stack of individual porous structure layers or is (ii) a continuous porous structure layer that is wound over a spiral holder to form said layers, and/or is wound around itself to form said layers. As a whole, each stack of porous structure layers provides a homogenous flow resistance and has a total thickness of 300 µm or more and 2000 µm or less. Further, in case of using one or two separate monolithic stabilized, non-ionic, hydrophilic porous structure(s), each of the same provides a homogenous flow resistance and has a total thickness of 300 µm or more and 2000 µm or less. In this context, the use of stacks of layers of a stabilized, non-ionic, hydrophilic porous structure is preferred, as this provides increased fluid flow homogeneity.

The porous structures used in the present invention can have a pore size of 1 to 5 µm, preferably of 2 to 4 µm, more preferably 2 to 3 µm, more preferably 2.5 to 3 µm. Further, each of said porous structure layers can have a thickness of 100 to 300 µm, preferably 150 to 300 µm, more preferably 180 to 270 µm, more preferably 200 to 250 µm, more preferably 210 to 240 µm, such as e.g., 220 or 230 µm.

Further, for lab scale use, at least the uppermost layer of said stack(s) of porous structure layers, *i.e.,* the first layer seen from an upstream-to-downstream direction, preferably all of the porous structure layers, as well as the monolithic porous structures, can have an accessible diameter, *i.e.,* a diameter of the maximum area that can be contacted with a fluid (e.g. a feed solution) provided to the filtration module, of more than 25 mm, preferably of at least 26 mm, wherein materials having an accessible diameter of 50 mm are particularly preferred. Accordingly, the layers or monoliths can have an accessible surface area, *i*.*e*., the maximum area that can be contacted with a fluid provided to the filtration module, of more than 4.91 cm², preferably of at least 5.31 cm², wherein materials having an accessible surface area of 19.6 cm² are particularly preferred. In other embodiments, the first layer seen from an upstream-to-downstream direction, preferably all of the porous structure layers, as well as the monolithic porous structures, can have an accessible diameter, *i*.*e*., a diameter of the maximum area that can be contacted with a fluid (e.g. a feed solution) provided to the filtration module, of from 10 to 293 mm, preferably 10 to 60 mm. The use of axial modules is restricted to a diameter of 293 mm. Larger diameters are technically not feasible because pressure increases with increasing membrane area of such a module design. With larger diameters, the pressures would be too high. Moreover, the flow distribution for such high diameters gets difficult, therefore capsule or cassette designs are preferred for scale-up.

For capsule designs, the incident flow area, *i*.*e*., the accessible surface area as defined above, can range between 6 cm² and 0.65 m². Further, cassette modules can have an incident flow area of 0.2 m² up to 11 m² with respective cassette module holders and an optimized cassette design. For example, to purify a 200 L LV feed solution of the type described in the method section hereinafter, an incident flow area of 3.84 m² is necessary (this corresponds to 19 non-optimized standard Sartobind cassettes).

Types of porous structures that can be used in the context of the present invention are not particularly limited, provided they have a non-ionic, hydrophilic surface. In preferred embodiments, the porous structure is a membrane or a monolith, wherein membranes are preferred. Any membrane or monolith structure with a hydrophilic (e.g., modified) surface can be used, e.g., polyethersulfone, polysulfone, polyvinylidene fluoride (PVDF), or polyamide. Respective membranes and monoliths are known in the art. Preferred membranes in this respect include stabilized cellulose membranes as known in the art (e.g. Sartorius Hydrosart^{®} membranes). In case where monolithic porous structures are used, the total thickness of the same is preferably 300 µm or more and 500 µm or less.

The filtration modules used in the methods of the present invention further comprise a housing as defined above. Respective housings can be designed in a way to give the filtration module used in the methods of the present invention the form of an axial flow module, a capsule module, or a cassette module, as known in the art.

Thus, in specific embodiments in which the filtration module is in the form of an axial flow module, the housing as defined above is a housing encasing one stack of porous structure layers, said housing comprising an upper lid portion including a fluid inlet and a lower table portion including a fluid outlet, wherein the lid portion and the table portion are firmly connected to each other in a fluid-tight and pressure-tight manner, and wherein the accessible dimensions of said stack of porous structure layers correspond to the inner dimensions of said housing, so that fluid entering the axial flow module through the fluid inlet has to pass through said stack of porous structure layers in order to exit the axial flow module through the fluid outlet, wherein the overall direction of fluid flow through the axial flow module is perpendicular to said stack of porous structure layers. In preferred embodiments in this respect, the inner surface of the upper lid portion of the housing and/or the inner surface of the lower table portion of the housing, preferably both, comprises radial and circular distribution channels to distribute the feed solution evenly over the accessible surface area of the stack of porous structure layers and to collect the fluid to the fluid outlet. In specific embodiments, the inner surface of the upper lid portion of the housing and/or the inner surface of the lower table portion of the housing, preferably both, comprises 8 radial distribution channels and 20 circular distribution channels. In preferred embodiments, said stack of porous structure layers and said housing are circular in shape. Further, in preferred embodiments, the axial flow module is provided in a multi-use stainless steel holder or in a single-use overmolded device, wherein the latter is preferred. In this context, the term "the accessible dimensions of the stack of porous structure layers correspond to the inner dimensions of the housing" as used herein refers to the situation where the inner lumen of the housing in the planar directions of the porous structure layers is entirely taken up by the stack of porous structure layers. This covers e.g., configurations in which a stack of porous structure layers has a larger diameter fitting into the inner dimensions of the housing, but the accessible diameter of said stack is smaller after the overmolding of the housing due to the clamping edge.

In this respect, in specific embodiments, the method of the present invention is a method for the purification of viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, by filtration-supported polyalkylene glycol precipitation, comprising the steps of:
(a) providing an axial flow module, said axial flow module comprising:
   (i) one stack of 2 to 9 layers of a stabilized, non-ionic, hydrophilic porous structure, said layers being deposited on top of each other, wherein said stack provides a homogenous flow resistance and has a total thickness of 300 µm or more and 2000 µm or less, and
   (ii) a housing encasing said stack of porous structure layers, said housing comprising an upper lid portion including a fluid inlet and a lower table portion including a fluid outlet, wherein the lid portion and the table portion are firmly connected to each other in a fluid-tight and pressure-tight manner, and wherein the accessible dimensions of said stack of porous structure layers correspond to the inner dimensions of said housing, so that fluid entering the axial flow module through the fluid inlet has to pass through said stack of porous structure layers in order to exit the axial flow module through the fluid outlet, wherein the overall direction of fluid flow through the axial flow module is perpendicular to said stack of porous structure layers,
(b) providing a feed solution, containing said viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, to said axial flow module via said fluid inlet at a surface area-dependent flow rate of at least 1.0 mL per minute per cm² of accessible surface area of the first layer of said stack of layers of a porous structure (1.0 min⁻¹·cm⁻²), whereby the viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, adhere to the porous structure, and
(c) eluting the viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, from said porous structure and thereby harvesting purified viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more.

In other specific embodiments in which the filtration module is in the form of a capsule module, the housing as defined above is a housing encasing one stack of porous structure layers, said housing comprising a fluid inlet from which the fluid flows into an external fluid channel from which the fluid passes through the stack of porous structure layers to an internal fluid channel which is connected to a fluid outlet, wherein the stack of porous structure layers is firmly connected to the external and internal flow channel in a fluid-tight and pressure-tight manner, and wherein the accessible dimensions of said stack of porous structure layers correspond to the inner dimensions of said housing, so that fluid entering the capsule module through the fluid inlet has to pass through said stack of porous structure layers in order to exit the capsule module through the fluid outlet, wherein the overall direction of fluid flow through the capsule module is radial to said stack of porous structure layers. In such capsule modules, the stack of porous structure layers is preferably rolled up or the capsules have the porous structure layers incorporated in a pleating technology. Within these capsule modules, the feed flows radially from the external fluid channel through the porous structure layers to the internal fluid channel. Further, the porous structure layers can be rolled, pleated, or folded porous structure layers around an internal fluid channel. Another possible module design is a spiral wound module. For the rolled-up porous structure layers, a stack of 2 to 9 layers of a stabilized, non-ionic, hydrophilic porous structure is provided, said layers being rolled up around an internal fluid channel, wherein said stack provides a homogenous flow resistance and has a total thickness of 300 µm or more and 2000 µm or less. A fleece material can be incorporated additionally. Pleated, folded, or other porous structure arrangements are possible, which should result in a similar thickness as the rolled-up version.

In other specific embodiments in which the filtration module is in the form of a cassette module, the housing as defined above is a housing encasing two separate stacks of porous structure layers or two separate monolithic stabilized, non-ionic, hydrophilic porous structures, said housing comprising a fluid inlet from which the fluid flows to into a central inlet flow channel, wherein the fluid enters on top between said two stacks or said two monolithic porous structures and passes through the stacks of porous structure layers or through the monolithic porous structures to outside (downstream) channels which are connected to a fluid outlet, wherein the two stacks of porous structure layers or the two monolithic porous structures are firmly connected to the central inlet flow channel and the outside channels in a fluid-tight and pressure-tight manner, and wherein the accessible dimensions of said stack of porous structure layers or said monolithic porous structures correspond to the inner dimensions of said housing, so that fluid entering the cassette module through the fluid inlet has to pass through said two stacks of porous structure layers or through said monolithic porous structures in order to exit the cassette module through the fluid outlet, wherein the overall direction of fluid flow through the cassette module is perpendicular to said stack of porous structure layers or said monolithic porous structures.

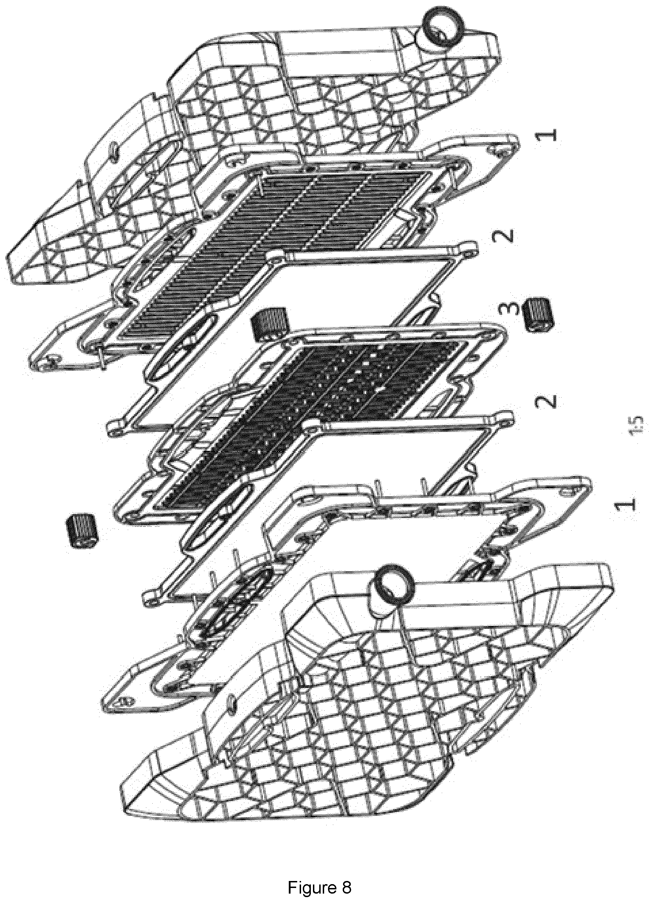

Current cassette module designs feature stacks of 15 membranes for membrane adsorber chromatography resulting in a bed height of 4 mm. The filtration area is 220 mm × 470 mm. The channel height distributing the sample before filtration is >5 mm and the combined filtrate channel height is >4 mm resulting in a total dead volume of 1600 mL for a combined filtration area of 0.2 m². Different from the chromatography needs, a PEG filtration device according to the present invention in the form of a cassette module as described above uses 2 to 9, preferably 3 to 5 membrane layers resulting in a thickness of 0.5 to 1 mm. More than one layer is necessary in order to achieve the same flux levels across the full area. For the intended process, the full filtration area is preferably addressed simultaneously. In order to achieve that, dead volume optimization and flow optimization are preferred. In particular, besides cutting the filter stack(s) (Fig. 8, (2)) thickness from 4 mm to 1 mm, the distribution plates (Fig. 8, (1)) upstream can be reduced in channel height to < 2,5 mm and the filtrate spacer (Fig. 8 (3)) to < 2,5 mm.

In step (b) of the methods of the present invention, a feed solution is provided to said flow module via said fluid inlet at a surface area-dependent flow rate of at least 1.0 mL per minute per cm² of accessible surface area of the first layer of said stack(s) of layers of a porous structure or of the monolithic porous structure(s) (1.0 mL·min⁻¹·cm⁻²), preferably at least 1.1 mL per minute per cm² of accessible surface area, more preferably at least 1.2 mL per minute per cm² of accessible surface area, more preferably at least 1.3 mL per minute per cm² of accessible surface area, more preferably at least 1.4 mL per minute per cm² of accessible surface area. Of note, there is no general specific upper limit for the surface area-dependent flow rate. However, technical limitations (e.g. with respect to the maximum pressure) lead to an effective upper limit depending on the specifications of the porous structure and the maximum pressure of the housing module. In specific embodiments, the surface area-dependent flow rate can be in a range of 1.0 to 3.5 mL per minute per cm² of accessible surface area (1.0 to 3.5 mL·min⁻¹·cm⁻²), preferably of 1.1 to 2.2 mL per minute per cm² of accessible surface area (1.1 to 2.2 mL·min⁻¹·cm⁻²), more preferably of 1.1 to 1.6 mL per minute per cm² of accessible surface area (1.1 to 1.6 mL·min⁻¹·cm⁻²). In specific embodiments, a flow rate of 1.426 mL·min⁻¹·cm⁻² is used. In this context, the above surface area-dependent flow rate is based on the accessible surface area of the first (i.e., uppermost / most upstream seen from an upstream-to-downstream direction) layer of the stack(s) of porous structure layers, or of the monolithic porous structure(s), not on the total accessible area. Thus, by way of example a stack of two porous structure layers, each having an accessible surface area of e.g. 5.31 cm² and a stack of nine porous structure layers, each having an accessible surface area of 5.31 cm², both have the same accessible surface area of the uppermost membrane of said stack of membranes of 5.31 cm².

In preferred embodiments, prior to step (b) of the methods of the present invention, the feed solution is mixed with a buffer in a ratio of buffer: feed solution of 4:1 to 1;4, preferably of 2:1 to 1:2, more preferably of 1.5:1 to 1:1.5, more preferably 1.2:1 to 1 :1.2. In specific embodiments, the virus- or virus-like particle-containing feed solution is mixed with a buffer in a ratio of buffer: feed solution of 1:1. Mixing is preferably effected by dynamic in-line mixing.

Respective buffers to be used in this respect can be selected from buffers providing an optimum environment for the viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, to be purified, wherein such buffers are known in the art. Of note, respective buffers compositions can be advantageously used throughout the methods of the present invention. In preferred embodiments, said buffer contains a polyalkylene glycol having two or three carbon atoms in its repeating unit, wherein the polyalkylene glycol is preferably polyethylene glycol (PEG), preferably a PEG having a molar mass of between 400 and 12000 g/mol, preferably between 2000 and 12000 g/mol, more preferably between 3000 and 8000 g/mol. In specific embodiments, PEG having a molar mass of 4000 g/mol (PEG 4000) is used. Preferably, the buffer contains the polyalkylene glycol or PEG in an amount of 4 to 20 wt.-% final polyalkylene glycol or PEG concentration after mixing with the feed solution, preferably 5 to 17.5 wt.-%, more preferably 7.5 to 15 wt.-%, more preferably 10 to 15 wt.-%. In particular embodiments, the buffer contains the polyalkylene glycol or PEG in an amount of 25 wt.-%, the mixing ratio of buffer : feed solution is 1:1, and, accordingly, the amount of polyalkylene glycol or PEG in the feed solution after mixing of the buffer with the feed solution is 12.5 wt.-%. Other PEG sizes can be used with adapted concentrations reflecting their molecular mass. The higher the molecular mass of PEG the smaller the needed PEG concentration.

According to the present invention, the feed solution is provided to the filtration module in step (b) of the methods of the present invention in a downflow flow direction, *i.e.,* in a direction from the fluid inlet towards the fluid outlet.

Elution in step (c) of the methods of the present invention uses an elution buffer not containing any polyalkylene glycol or PEG, or an elution buffer containing any polyalkylene glycol or PEG in amount that is lower than the amount used in the buffer used for mixing prior to step (b). In this context, a lower amount is preferably 80% or less, more preferably 50% or less, more preferably 20% or less, most preferably 0%, with respect to the polyalkylene or PEG concentration in the buffer used for mixing prior to step (b). The higher the concentration of polyalkylene glycol or PEG in the elution buffer, the broader the elution peak will be, resulting in lower target product concentrations in the elution. Of note, a gradient elution as known in the art can be used in this step, wherein the amount of any polyalkylene glycol or PEG is gradually lowered, starting from the amount of any polyalkylene glycol or PEG used in the buffer used for mixing prior to step (b) down to a buffer not containing any polyalkylene glycol or PEG. Nevertheless, preferably the elution in step (c) of the methods of the present invention uses an elution buffer not containing any polyalkylene glycol or PEG. Thus, in specific embodiments, the elution buffer is the same buffer used for mixing prior to step (b), but not containing any polyalkylene glycol or PEG. Elution can be performed either in a downflow flow direction or in an upflow flow direction, *i.e.,* in a direction from the fluid outlet (which in this case effectively acts as a fluid inlet) to the fluid inlet (which in this case effectively acts as a fluid outlet), wherein the latter case is preferred.

As indicated above, the methods of the present invention are based on SXC virus purification processes known in the art. As such, the methods of the present invention can contain an equilibration step prior to step (b), as well as one or more washing steps after equilibration and prior to step (b), and/or after step (b) and prior to step (c).

Viruses that can be purified in the methods of the present invention are not particularly limited and include in particular large, enveloped viruses known in the art. Preferably, the virus to be purified in the methods of the present invention is selected from the group consisting of lentiviruses, baculoviruses, Orf virus, adeno-associated viruses (AAV), influenza viruses (such as influenza A virus), yellow fever viruses, human papillomaviruses, vaccinia viruses, adenoviruses, hepatitis viruses, polioviruses, rabies viruses, rotaviruses, rubellaviruses, and Zika viruses, as well as respective virus-like particles. In specific embodiments, the virus is a lentivirus. In other specific embodiments, the virus is an AAV.

In this context, as used herein, the terms "virus" and "viral vector" are used synonymously.

Further, virus-like particles that can be purified in the methods of the present invention are not particularly limited and include virus particles that do not contain nucleic acids. Furthermore, virus-like particles include viroids, virusoids and prions.

Further, extracellular vesicles (such as exosomes or microvesicles), lipid nanoparticles, proteins or proteins complexes, nucleic acids, protein-nucleic acid complexes, each having a diameter of 5 nm or more, can be purified with the present invention. Potential protein targets could be antibodies, antibody-drug conjugates (ADC), bi- or multispecific molecules, membrane proteins, and membrane protein complexes, in particular large membrane protein complexes. Preferably, proteins and molecules that can be purified in the present invention are globular proteins and globular molecules, respectively.

As described herein, the methods of the present invention are filtration-supported polyalkylene glycol precipitation methods. However, in related aspects, precipitation in such methods could also be effected by way of ammonium sulfate precipitation, precipitation using polymers other than polyalkylene glycols, polyelectrolyte precipitation, or pH shift precipitation, as known in the art.

In a second aspect, the present invention relates to filtration module, comprising:
(a-α) one stack or two separate stacks of 2 to 9 layers of a stabilized, non-ionic, hydrophilic porous structure, said layers being deposited on top of each other, wherein each of said stack(s) provide(s) a homogenous flow resistance and has a total thickness of 300 µm or more and 2000 µm or less, or
(a-β) one monolithic stabilized, non-ionic, hydrophilic porous structure, or two separate monolithic stabilized, non-ionic, hydrophilic porous structures, wherein each of said porous structure(s) provide(s) a homogenous flow resistance and has a total thickness of 300 µm or more and 2000 µm or less, and
(b) a housing encasing said stack(s) of porous structure layers, said housing comprising a fluid inlet and a fluid outlet, wherein fluid entering the filtration module through the fluid inlet has to pass through said stack(s) of porous structure layers in order to exit the filtration module through the fluid outlet, and wherein the housing is adapted to distribute the feed solution entering the housing via the fluid inlet evenly over the entire accessible surface area of the first layer of said stack(s) of porous structure layers.

The flow module according to the present invention is as defined in the above first aspect of the present invention.

In particular, all definitions of the flow module provided in the context of the methods of the present invention according to the first aspect of said invention equally apply to the flow module as such according to the second aspect of the present invention.

Thus, in specific embodiments, the flow module of the present invention is an axial flow module, comprising:
(a) one stack of 2 to 9 layers of a stabilized, non-ionic, hydrophilic porous structure, said layers being deposited on top of each other, wherein said stack provides a homogenous flow resistance and has a total thickness of 300 µm or more and 2000 µm or less, and
(b) a housing encasing said stack of porous structure layers, said housing comprising an upper lid portion including a fluid inlet and a lower table portion including a fluid outlet, wherein the lid portion and the table portion are firmly connected to each other in a fluid-tight and pressure-tight manner, and wherein the accessible dimensions of said stack of porous structure layers correspond to the inner dimensions of said housing, so that fluid entering the axial flow module through the fluid inlet has to pass through said stack of porous structure layers in order to exit the axial flow module through the fluid outlet, wherein the overall direction of fluid flow through the axial flow module is perpendicular to said stack of porous structure layers.

In such embodiments, it is preferred that the inner surface of the upper lid portion of the housing and/or the inner surface of the lower table portion of the housing, preferably both, comprises radial and circular distribution channels to distribute the virus- or virus-like particle-containing feed solution evenly over the accessible membrane surface area of the stack of membranes and to collect the fluid to the fluid outlet. In specific embodiments, the inner surface of the upper lid portion of the housing and/or the inner surface of the lower table portion of the housing, preferably both, comprises 8 radial distribution channels and 20 circular distribution channels.

In other specific embodiments, the flow module of the present invention is a capsule module, comprising:
(a) one stack of 2 to 9 layers of a stabilized, non-ionic, hydrophilic porous structure, said layers being deposited on top of each other, wherein said stack provides a homogenous flow resistance and has a total thickness of 300 µm or more and 2000 µm or less, and
(b) a housing encasing said stack of porous structure layers, said housing comprising a fluid inlet from which the fluid flows into an external fluid channel from which the fluid passes through the stack of porous structure layers to an internal fluid channel which is connected to a fluid outlet, wherein the stack of porous structure layers is firmly connected to the external and internal flow channel in a fluid-tight and pressure-tight manner, and wherein the accessible dimensions of said stack of porous structure layers correspond to the inner dimensions of said housing, so that fluid entering the capsule module through the fluid inlet has to pass through said stack of porous structure layers in order to exit the capsule module through the fluid outlet, wherein the overall direction of fluid flow through the capsule module is radial to said stack of porous structure layers.

In other specific embodiments, the flow module of the present invention is a cassette module, comprising:
(a-α) one stack or two separate stacks of 2 to 9 layers of a stabilized, non-ionic, hydrophilic porous structure, said layers being deposited on top of each other, wherein each of said stack(s) provide(s) a homogenous flow resistance and has a total thickness of 300 µm or more and 2000 µm or less, or
(a-β) one monolithic stabilized, non-ionic, hydrophilic porous structure, or two separate monolithic stabilized, non-ionic, hydrophilic porous structures, wherein each of said porous structure(s) provide(s) a homogenous flow resistance and has a total thickness of 300 µm or more and 2000 µm or less, and
(b) a housing encasing said two separate stacks of porous structure layers or two separate monolithic stabilized, non-ionic, hydrophilic porous structures, said housing comprising a fluid inlet from which the fluid flows to into a central inlet flow channel, wherein the fluid enters on top between said two stacks or said two monolithic porous structures and passes through the stacks of porous structure layers or through the monolithic porous structures to outside (downstream) channels which are connected to a fluid outlet, wherein the two stacks of porous structure layers or the two monolithic porous structures are firmly connected to the central inlet flow channel and the outside channels in a fluid-tight and pressure-tight manner, and wherein the accessible dimensions of said stack of porous structure layers or said monolithic porous structures correspond to the inner dimensions of said housing, so that fluid entering the cassette module through the fluid inlet has to pass through said two stacks of porous structure layers or through said monolithic porous structures in order to exit the cassette module through the fluid outlet, wherein the overall direction of fluid flow through the cassette module is perpendicular to said stack of porous structure layers or said monolithic porous structures.

As used herein, the term "comprising"/"comprises" expressly includes the terms "consisting essentially of'/"consists essentially of" and "consisting of'/"consists of", *i.e.,* all of said terms are interchangeable with each other herein.

In the present invention, the LV location on the stationary phase of a small scale-device was shown. Based on these results, an intermediate scale-up approach was investigated with a device of 4-fold membrane surface area. This revealed the critical aspect of a scaled flow rate, as well as the importance of module design for successful LV recovery using filtration-supported polyalkylene glycol precipitation in a scale-up format.

In this context, it is noted that if SXC is considered as a chromatography method, the entire column is assumed to be used in depth and therefore scaling is difficult on this basis. However, the present invention has advantageously shown that there is no use in depth. Accordingly, the present invention speaks of filtration-supported polyalkylene glycol precipitation. This is in difference to the commonly known SXC process where the whole depth of the stationary phase is used instead of just the membrane layer to a depth of about 300 µm at most.

Specifically, the scale-up of lentiviral vector purification by SXC with an axial flow membrane module was approached. Visualization of the LV captured on the membrane during SXC showed that this is a surface-oriented process using mainly the upper membrane layers. When transferring the SXC process to a module with a 4-fold larger area, it became apparent that the scaling of the flow rate is a critical factor and must be related to the membrane area of the first (most upstream) membrane layer. An infectious lentiviral vector recovery of 73% and removal of proteins and dsDNA of 77% and 63%, respectively, were obtained with the optimized conditions and the overmolded membrane devices. Housing prototypes of the modules were constructed, and LV capture on the membrane and SXC performance were analyzed, with findings demonstrating that the flow rate in relation to the membrane area of the first membrane layer, as well as the uniform usage of the membrane, are of importance in this respect.

Thus, the present invention advantageously identified the adsorption of viruses or virus-like particles to stabilized cellulose membranes during SXC as a largely surface-dependent process. This led to the surprising finding that any up-scaling of SXC for the purification of viruses or virus-like particles requires up-scaling of the flow rate in relation to the accessible membrane area of the uppermost membrane of the membrane stack, as well as the optimization of fluid distribution to the membrane.

Module designs offering larger membrane surface areas with increasing demand in capacity, due to the surface-related capture process, scale-up results in a higher flow facing the proportional larger surface area but the processing time, as well as the transmembrane pressure remains constant.

The figures show:
Figure 1:
   SXC membrane devices.
   MA15 (25 mm membrane diameter) in an (A) stainless-steel holder or (B) overmolded. (C) The inner structure of the MA15 lid and table with radial and circular distribution channels and (D) a cross-sectional view of an SXC membrane device. MA100 (50 mm membrane diameter) in an (E) stainless-steel holder or (F) overmolded. (G) Inner support geometry of the MA100 lid and (H) structure of the MA100 table with radial and circular distribution channels.
Figure 2:
   Membrane layers of an MA15 device after loading lentiviral vectors labeled with anti-VSV-G Alexa Fluor^{®} 546 antibody.
   Membrane layers were separated after the (A) loading and wash step of steric exclusion chromatography or (B) after the elution step. A membrane that was not incorporated into the device serves as a negative control.
Figure 3:
   Identification of critical process parameters to achieve a high infectious LV recovery.
   (A) Infectious LV recovery for different flow rates between 3 and 9 mL·min⁻¹. (B) Infectious LV recoveries of MA15 and MA100 with the same surface area-dependent flow rate of 1.426 mL·min⁻¹·cm-², MA15 N=6, MA100 N=11. (C) Infectious LV recovery for internal and externally mixed LV-PEG-solution, N=3. Data in B&C represents mean ± standard deviation. P-values are indicated as follows: * p ≤ 0.05, ** p ≤ 0.01, n.s. - not significant.
Figure 4:
   Impurity removal by steric exclusion chromatography.
   (A) Protein and dsDNA removal using MA15 and MA100 devices with the same surface area-dependent flow rate of 1.426 mL·min⁻¹·cm⁻², MA15 N=6, MA100 N=11. (B) SDS-PAGE gel with silver staining of SXC fractions; 1 - Marker, 2 - Loading material, 3 - Flow through, 4 - Wash, 5 - Elution. Protein bands refer to VSV-G envelope protein, reverse transcriptase (RT) subunit p51, and p66, integrase (INT), capsid (CA), and matrix (MA). Data in A represents mean ± standard deviation.
Figure 5:
   Identification of loading capacity.
   Infectious LV recovery for loading (A) 200 mL or (D) 700 mL. Phase contrast image merged with green fluorescence channel image of HEK293T cells after incubation with a (B) flow through fraction or (C) elution fraction. Main effect plots of infectious (E) LV recovery and (F) LV particle recovery for different loading volumes.
Figure 6:
   Membrane layers of different MA100 device designs after loading lentiviral vectors labeled with anti-VSV-G Alexa Fluor^{®} 546 antibody.
   Membrane layers were separated after the loading and wash step of steric exclusion chromatography. The lid and table configurations of the housing are indicated on the left with (A) standard configuration, (B) reversed configuration, and (C) prototype configuration.
Figure 7:
   LV recoveries for different MA100 housing configurations.
   Infectious and particle LV recovery for (A) the standard and reversed configuration and (B) the standard and prototype configuration of the MA100 housing operated at 1.426 mL·min⁻¹·cm⁻², N=3 each. Data represent mean ± standard deviation.
Figure 8:
   Standard cassette design.
   Standard cassette design of existing cassette modules. The module consists of distribution plates (1), membrane stack(s) (2), and filtrate spacers (3). On the basis of this standard configuration the optimized cassette design according to the present invention is devised.
Figure 9:
   Surface-area specific flow rates.
   (A) Particle and (B) infectious LV recovery, as well as (C) dsDNA and (D) protein removal of SXC experiments plotted against different surface area-specific flow rates for different module sizes. Replicates for each device and flow rate are as follows: PP15 N=3 for all flow rates; MA15 N=3 for all flow rates; MA100 for 1.43 mL·min⁻¹·cm⁻² N=11, for all other flow rates N=3; and 5" capsule N=1.

The present invention will be further illustrated by the following examples without being limited thereto.

### Examples

### Material and methods:

### Lentiviral vector production, harvest, and clarification

Third-generation lentiviral vectors were produced by transient transfection of suspension HEK293T/17 SF cells (ACS-4500, ATCC) with four plasmids in a UniVessel^{®} 10 L bioreactor operated by a BIOSTAT^{®} B-DCU (Sartorius). The pH electrode was calibrated, and the vessel was assembled (containing a 2×3 blade segment impeller, ring-up sparger) and filled with 30% of its volume with water. The bioreactor was autoclaved at 121°C. After autoclaving, the bioreactor was emptied and filled with 80% of the final volume with FreeStyle medium (Thermo Fisher Scientific) + 0.0002% Antifoam C (Sigma Aldrich) + 1× insulin-transferrin-selenium (Thermo Fisher Scientific). The bioreactor was connected to the BIOSTAT^{®}, the DO probe was calibrated, and the pH electrode was re-calibrated. Cultivation setpoints were: stirrer speed 202 rpm, 30 % DO, 37 °C, pH 7.1. The bioreactor was left overnight to adjust pH and pO₂. The next day, the bioreactor was inoculated with 9% of the final bioreactor volume with a final viable cell density of 0.3×10⁶ cells·mL⁻¹. After inoculation, and once daily onwards, the bioreactor was sampled for viable cell density and viability determination with the Cedex HiRes (Roche) and offline pH measurement. The pH probe was re-calibrated when a difference of > 0.1 was detected between the externally and internally measured pH. Three days after inoculation, transfection was performed. 0.5 mg total plasmid DNA was used per liter of final culture volume in a mass ratio of 5:2.5:1:1 (pALD-Lenti-GFP : pALD-GagPol : pALD-VSV-G : pALD-REV1; Aldevron) and was prepared in FreeStyle medium without additives. In a separate flask, 4 mL, PElpro per mg of total plasmid DNA was diluted in FreeStyle (in 5% of the final bioreactor volume each). The two solutions were mixed and, after incubation for 15 min, added to the bioreactor. 18 h after transfection, the following reagents were added to the bioreactor: anti-clumping agent (1:500 (v/v)), the enhancer sodium butyrate (final concentration of 10 mM), and 1 mL of 2% Antifoam C. A nuclease treatment for nucleic acid digestion was performed with 10 U·mL⁻¹ DENARASE^{®} (c-Lecta) and 2 mM MgCl₂ (final concentrations) directly in the bioreactor for 1 h at 37 °C. After nucleic acid digestion, the cell culture broth (which contained the lentiviral vector) was clarified using Sartoclear Dynamics^{®} Lab V50 (0.45 µm polyethersulfone membrane version) with 5 g/L diatomaceous earth (Sartorius). The lentiviral vector was aliquoted and stored at -80 °C.

### Membrane and housing

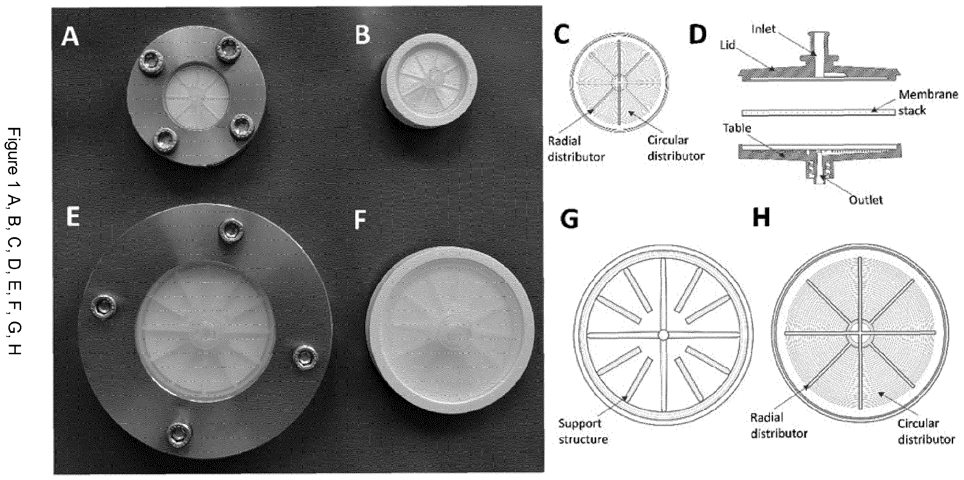

A stabilized cellulose membrane Hydrosart^{®} 10242 (Sartorius) was used as a stationary phase. The membrane lot used in this study has a thickness of 220 µm per layer and a mean flow pore size of 2.5-3 µm. Stacks of 5 membrane layers with a diameter of 57 mm were incorporated into an MA100 polypropylene module and overmolded with an Arburg 221-75-350 injection molding machine. The final chromatography module has an accessible membrane diameter of 50 mm, resulting in an accessible membrane surface area of 19.63 cm² per layer. The recommended maximum pressure for this device is 0.6 MPa. For comparison, the small-scale SXC device MA15 with the same membrane (5 layers) incorporated was used. Moreover, for further scaling experiments an axial PP15 device with 16 mm diameter and a 5" capsule were used. For lentiviral vector visualization experiments, the membranes and housing were incorporated into a stainless-steel holder to access membranes easily for visualization. MA15 and MA100 membrane devices are shown in Fig. 1, and specifications of all modules are listed in Table 1. The MA15 has ¼ of the surface area and membrane volume of the MA100 device.

The membrane housing of the MA100 used in this study has a lid and a table with distinct geometries. The lid has a coarse structure with thicker bridges that prevent the membrane from pressing tightly against it and is intended to give the liquid room to spread (Fig. 1 G). The table has 8 radial distribution channels and 20 circular distribution channels that collect the fluid to the outlet (Fig. 1 H). The PP15 and MA15 devices have the same distribution channel geometry in the lid and table (Fig. 1C).

**Table 1: Specifications for SXC chromatography with different device scales.**

| **Specification** | **PP15** | **MA15** | **MA100** | **5" capsule** |
|---|---|---|---|---|
| Equilibration volume | 8 mL | 20 mL | 80 mL | 2 L |
| Loading volume | 20 mL | 40 mL | 160 mL or higher | 1.96 L |
| Loaded LV volume | 10 mL | 25 mL | 80 mL or higher | 0.98 L |
| Wash volume | 8 mL | 15 mL | 60 mL | 2 L |
| Elution volume | 10 mL | 20 mL | 80 mL or higher | 0.98 L |
| Device geometry | axial | axial | axial | radial |
| Diameter of axial devices | 16 mm | 25 mm | 50 mm | - |
| Surface area per layer | 2.01 cm² | 4.91 cm² | 19.63 cm² | 192 cm² |
| Membrane volume | 0.22 cm³ | 0.565 cm³ | 2.257 cm³ | 75 cm³ |
| Scaling factor | 1 | 2.5 | 10 | 98 |

### Chromatography setup and procedure

The chromatography system ÄKTA^{™} avant 150 (Cytiva Life Sciences) with inline UV (280 nm) and conductivity monitoring operated by UNICORN 7.1 software was used to purify the lentiviral vectors by SXC using the PP15, MA15, and MA100 module. For the large-scale SXC experiments with the 5" capsule of 4 mm bed height (Sartorius) a multi-use membrane chromatography system (MU RCC, Sartorius) was used with a PuraLev^{®} i30SU pump (Levitronix) installed inline operated at 600 rpm, which served as a dynamic mixer for the buffers and feed solution. All chemicals (Tris, hydrochloric acid (HCl), sodium chloride (NaCl), PEG 4000) were purchased from Carl Roth. Buffers were prepared in ultrapure water of Arium^{®} Pro (Sartorius). Two buffers were prepared: 1) 50 mM Tris-HCl buffer with 150 mM NaCl, pH 7.4 (A1), and 2) 25% PEG 4000 in 50 mM Tris-HCl, 150 mM NaCl pH 7.4 (B1). In the following, the buffers are referred to as Tris buffer and PEG buffer, respectively.

Chromatography runs with the MA15 device were performed as known in the art, and volumes for equilibration, loading, wash, and elution were adapted to the MA100 device as listed in Table 1.

On the day of the experiment, the LV sample was thawed in a water bath at 37 °C until only small ice clumps remained. The sample was then stored at 4 °C until use (30-60 min). The entire LV solution was used on the day of thawing. Different LV batches were used for different experiments; therefore, the respective titer of each LV sample is indicated in the Examples below. The LV solution was kept on ice during the experiments, and the fractions were collected and cooled at 4 °C. The MA100 membrane device was first equilibrated with the Tris buffer and the PEG buffer, which were mixed in-line at a ½ dilution. The PEG buffer with a concentration of 25% (w/v) PEG 4000 then reached a final PEG concentration of 12.5%. The LV sample (A2) was loaded by in-line mixing with the PEG buffer at a ½ dilution in downflow direction, if not indicated otherwise. The loading volume varied between experiments and is provided in the below Examples for each experiment. The membrane column was washed with Tris buffer and PEG buffer, which were mixed in-line at a ½ dilution. The LVs were eluted with Tris buffer in upflow direction. Fractions were aliquoted and stored at -80 °C for analysis. The flow rates, loading volume, and SXC membrane device design varied depending on the experiment and are mentioned in the respective Examples below. For a high flow rate (28 mL·min⁻¹), an open configuration of the chromatography system was used by manually collecting the fractions directly after the chromatography device without running through the whole system to reduce the pressure. A new membrane device was used for every run.

### Analytics:

### Infectious titer determination

The infectious LV titer was quantified with the Incucyte^{®} S3 live-cell analysis system (Sartorius). Adherent HEK293T cells (ACC 635, DSMZ) were infected with serially diluted LV samples, and GFP expression was measured by real-time imaging as known in the art, with the following modifications: no staining was performed, and transgene expression (GFP) was read out 48 h post-infection. Samples were analyzed in duplicates.

### Particle titer determination

The LV particle titer was quantified with an enzyme-linked immunosorbent assay (ELISA) using the QuickTiter^{™} Lentivirus titer kit (Cell Biolabs) that quantifies the p24 capsid protein. The absorbance was read at 450 nm with a FLUOstar Omega plate reader (BMG Labtech). The standard curve obtained was fitted by a second-degree polynomial. The p24 concentrations determined were converted into viral particle titers by assuming that 1.25 × 10⁷ LV particles contain 1 ng of p24, and 1 LV particle contains about 2000 molecules of p24.

### Total protein quantification

The total protein concentration was determined with the Pierce^{™} Coomassie Bradford protein assay kit (Thermo Fisher Scientific) according to the manufacturer's instructions. Standards and samples were analyzed in duplicates in transparent 96-well microtiter plates (Greiner Bio-one). The absorbance was read at 595 nm with a microplate reader. The standard curve obtained was fitted by linear regression.

### Total dsDNA quantification

The total dsDNA amount was quantified with the Quant-iT^{™} Pico-Green^{™} dsDNA assay (Thermo Fisher Scientific) according to the manufacturer's instructions. Standards and samples were analyzed in duplicates in black 96-well microplates (Corning). The samples were excited at 480 nm, and the fluorescence emission intensity was measured at 520 nm using a microplate reader. The standard curve obtained was fitted by linear regression.

### SDS-PAGE and Silver Staining

Proteins were fractionated by SDS-PAGE in 4-15% Mini-PROTEAN^{®} TGX Stain-Free protein gels (Bio-Rad). The SDS-PAGE was performed according to the manufacturer's instructions. Precision Plus protein standard (Bio-Rad) served as a marker. The gel was run at a constant voltage of 300 V for 15-20 min. Protein bands were visualized with a Pierce Silver Stain Kit (Thermo Fisher Scientific).

### Lentiviral vector visualization

Staining was performed to visualize the location of the LV on the membrane before and after elution. The LV sample was incubated for 1 h at 4 °C with a mouse monoclonal antibody to VSV-G (F-6) labeled with Alexa Fluor^{®} 546 (Santa Cruz Biotechnology) in a dilution of 1 :2000. Five layers of the Hydrosart^{®} membranes were placed between the table and lid of the chromatography device that is incorporated in a stainless-steel holder (Fig. 1 A and E). The screws were tightened to 3 Nm. The SXC run was performed as described above and stopped after the wash step before elution. The membranes were separated and visualized with a UVP ChemStudio (Analytik Jena) by applying the green light source (550 nm), the ethidium bromide filter, and an exposure time of 60 s. An untreated membrane layer that was not incorporated into the membrane holder device was used as a negative control.

### Statistical analysis:

The statistical significance of between-group differences was evaluated using an unpaired Student's t-test (two-tailed) with OriginPro^{®} 2021 (OriginLab). Where applicable, experiments were evaluated with MODDE Pro 13 (Sartorius).

### Example 1:

### Lentiviral vector visualization on a membrane

To date, it is unclear where the target product (in this case, the LV) is located on the membrane after loading by SXC, and the literature lacks studies of particle localization in the stationary phase during SXC. Visualizing LV on the stationary phase could contribute to the understanding of the SXC process and process requirements.

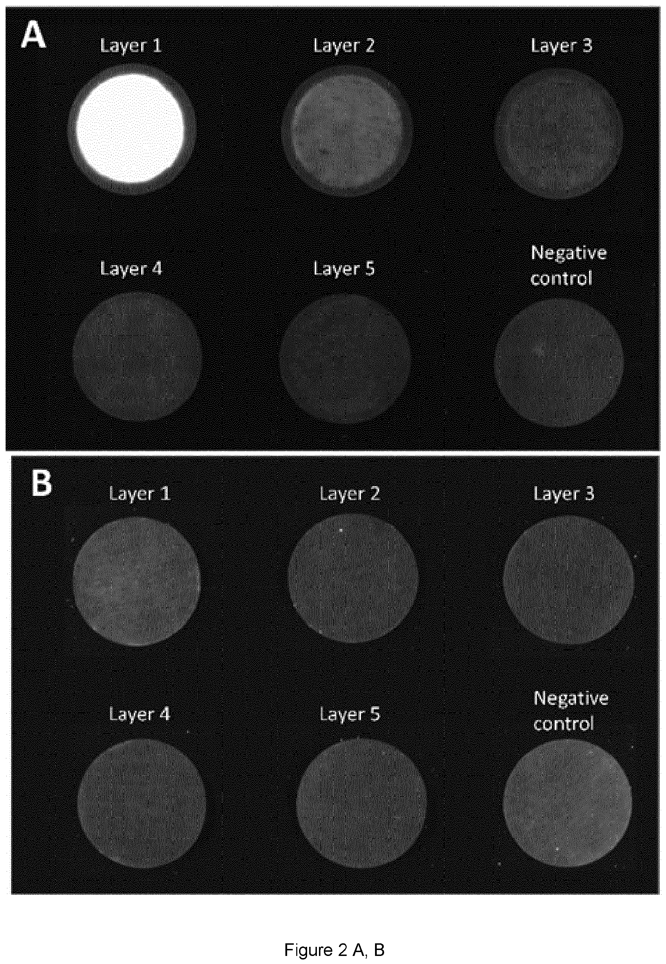

Lentiviral vectors were stained with an anti-VSV-G antibody labeled with Alexa Fluor^{®} 546 as described above. The labeled LV was loaded on the membrane that was incorporated into an MA15 housing and placed in a stainless-steel holder (Fig. 1 A). SXC was performed using a PEG buffer with a final PEG 4000 concentration of 12.5 % and a flow rate of 7 mL·min⁻¹. A volume of 50 mL was loaded, which corresponds to a volume of 25 mL LV solution with 1.5×10¹¹ virus particles per mL (VP·mL⁻¹). The SXC runs were stopped after the loading and wash step (Fig. 2 A) or after the elution step (Fig. 2 B) for optical visualization.

Fig. 2 shows the visualization of captured viral vectors on the stationary phase after loading by SXC. LV particles were mainly present on the first and second layers of the membrane. Some LV particles can be detected on layer 3, but no fluorescence was detected on layers 4 and 5. The particles are homogeneously distributed on the membrane layers. Only the clamping edge, which is not in contact with the liquid, is not stained accordingly. These findings indicate that with SXC, very few viral particles were captured in the depth of the unit. Therefore, using 15 layers - as often described in the literature - does not appear to offer any added value compared to 5 layers. Furthermore, column volume, which is specified for other classical chromatography devices, plays a minor role in the SXC method. Although it is a straightforward approach, visualization of the viral vectors on the membrane indicates that adding more membrane layers (thereby increasing the membrane volume) does not appear to be a valuable scaling method. The surface area of the first layer is a more important feature for SXC. It is assumed that once the first layer is saturated, the access to further layers is restrained, and a multilayer of LV particles is built, reducing the pore size. Hence, a pressure increase is observed during loading, as previously reported. After elution, no fluorescence was detected on the membrane, indicating that (almost) all LV were eluted.

### Example 2:

### identifying critical process parameters for SXC scale-up

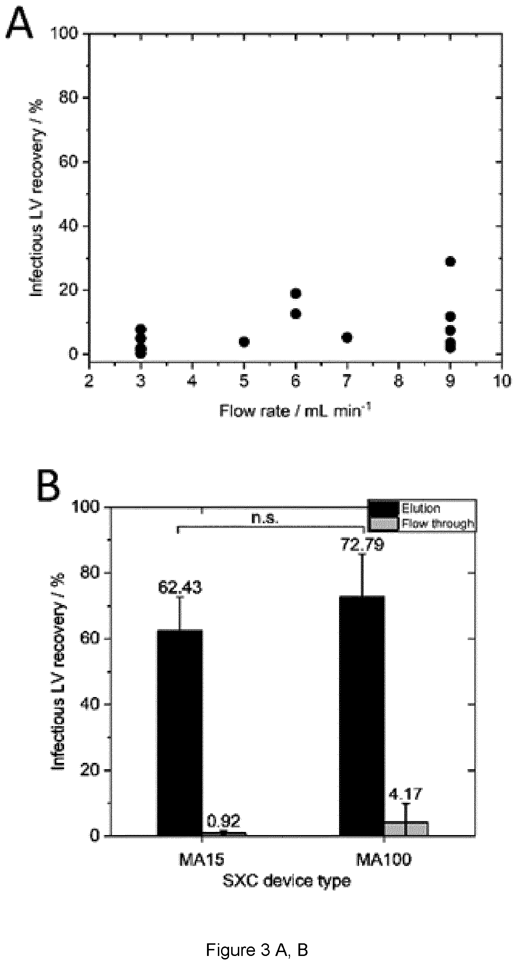

SXC has so far only been performed on a small scale using axial membrane devices with a diameter of up to 25 mm. By increasing the membrane surface area 4-fold, it was attempted to identify critical process parameters for successful scale-up purification of lentiviral vectors by SXC. Previous research with a small-scale MA15 device determined 12.5% PEG 4000 as an ideal buffer for the purification of LV with SXC. Therefore, this buffer composition was used and not further modified in the following experiments. In the same study using the MA15, an optimal flow rate of 6-7 mL·min⁻¹(tested flow rate range 3-9 mL·min⁻¹) was identified, achieving an infectious LV recovery above 80%. In a first attempt, flow rates between 3-9 mL·min⁻¹ were tested using the MA100 device and an LV batch with a titer of 1.25×10⁷ transducing units per mL (TU·mL⁻¹). Fig. 3 A shows that lower than expected infectious LV recoveries were observed. It was concluded that the optimal flow rate for the MA100 device is not within this range.

For membrane chromatography, the flow rate is typically given in membrane volumes per mL. As discussed in Example 1, scaling up by only increasing the membrane volume but not the surface area of the first membrane does not seem to be useful with respect to the surface-oriented capture of the vector particles. Hence, a specification of the flow rate per membrane surface area (of one layer) would be more reasonable than the flow rate per membrane volume. Given the dynamic depletion flocculation process of SXC, it was hypothesized that the flow rate is dependent on the surface area of one membrane layer. The previously determined optimal flow rate of 7 mL·min⁻¹ for the MA15 device corresponds to a surface area-dependent flow rate of 1.426 mL·min⁻¹·cm⁻². It was aimed to apply the same surface area-dependent flow rate for the MA100 device. As the membrane surface area of one layer is 4-times larger, a flow rate of 1.426 mL·min⁻¹·cm⁻² for the MA100 device corresponds to 28 mL·min⁻¹(Table 1).

However, the pressure limit was reached when applying 28 mL·min⁻¹ with the viscous PEG buffer. The UV cell and fractionator of the chromatography system contribute to the pressure. To circumvent this technical limitation, the chromatography system was opened after the column position and fractionated manually. This adjustment allowed to apply the flow rate of 1.426 mL·min⁻¹·cm⁻² (28 mL·min⁻¹) for the MA100 device. SXC runs were performed with an LV batch with a titer of 1.73×10⁷ TU·mL⁻¹ at 1.426 mL·min⁻¹·cm⁻² with the MA15 and MA100 device and no significant differences in infectious titer were detected (Fig. 3 B). The MA100 yielded an infectious LV recovery of 72.79 ± 12.92%. These results confirm the hypothesis that the flow rate must be scaled to the surface area of the membrane layer. The flow velocity through the stationary phase seems to be a decisive factor in the purification success. When the same flow rate in mL·min⁻¹ is applied to the MA100 device, the same feed is distributed to a larger surface area, and thus, to a higher number of pores compared to the MA15. Since the average pore diameter remains unchanged, the flow velocity inside the pores decreases and falls below the optimal flow velocity inside the stationary phase to achieve efficient LV capture during loading and release during elution.

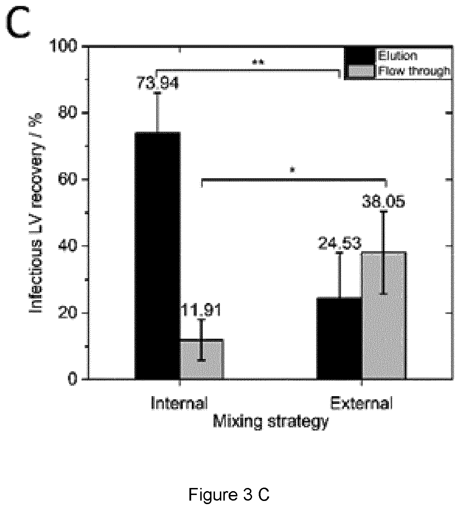

Internal and external mixing of the LV solution with PEG buffer was performed for the MA100 SXC runs as previously performed for the MA15 runs and as known in the art. Briefly, LV solution (titer of 1.64×10⁷ TU·mL⁻¹) was mixed with PEG buffer in a flask with a magnetic stirrer. After 1 h of incubation at 4 °C, the sample was loaded onto the membrane (external mixing) or by loading via pump A and pump B of the chromatography system with mixing in the dynamic mixer shortly before reaching the membrane device. The infectious LV recovery was significantly higher (p ≤ 0.01) when internal mixing was performed (Fig. 3 C) compared with external mixing (73.94 ± 12.13% and 24.53 ± 13.43%, respectively). These findings support previous results, in which the same effect was observed with an MA15 device. Moreover, a significantly higher amount of LV (38.05 ± 12.37%) was lost in the flow through (p ≤ 0.05) when LV was loaded after external mixing. The external mixing of PEG buffer with the LV solution and incubation could have led to LV aggregation as depletion interaction can occur not only between LV and the stationary phase during the SXC loading step but also of viral particles with one another. Forming aggregates, the free energy of the system is already reduced, leading to a less effective depletion interaction of the LV with the membrane and loss in the flow through. These observations underline the highly dynamic nature of this chromatography method, as already observed by the importance of the flow rate.

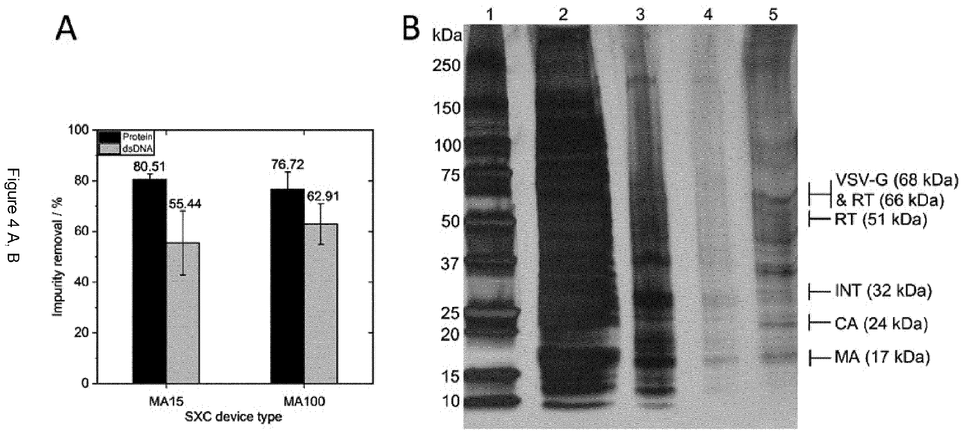

The protein and dsDNA removal using the MA15 and MA100 devices at the same surface area-dependent flow rate of 1.426 mL·min⁻¹·cm⁻² was next investigated (Fig. 4). The dsDNA and protein concentrations of the loading material and elution fractions are listed in Table 2. Overall, high removal of proteins (80.51 ± 2.22% for the MA15 and 76.72 ± 6.81% for the MA100) was observed. A silver-stained SDS-PAGE gel confirms the measurement, showing a high amount of protein contaminants in the loading material and the removal of the majority of the protein impurities in the flow through (Fig. 4 B). The elution fraction shows protein bands for the structural proteins of the lentiviral vector and little contaminating protein. The dsDNA removal was 55.44 ± 12.58% for the MA15 and 62.91 ± 8.06% for the MA100. These results demonstrate that comparable impurity removals are obtained for both device types. The effective removal of impurities derives from the pronounced size differences between the LV and the contaminating proteins and DNA.

**Table 2: Protein and dsDNA concentration in loading material and elution fraction for SXC chromatography with the MA15 or MA100 device.**

| **Device** | **Protein in loading material [per µg·mL⁻¹]** | **Protein in elution fraction [per µg·mL⁻¹]** | **dsDNA in loading material [per µg·mL⁻¹]** | **dsDNA in elution fraction [per µg·mL⁻¹]** |
|---|---|---|---|---|
| MA15 | 268.45 ± 10.06 | 52.26 ± 5.65 | 283.49 ± 87.07 | 55.44 ± 12.58 |
| MA100 | 253.38 ± 39.95 | 57.38 ± 15.15 | 356.35 ± 81.43 | 62.91 ± 8.06 |

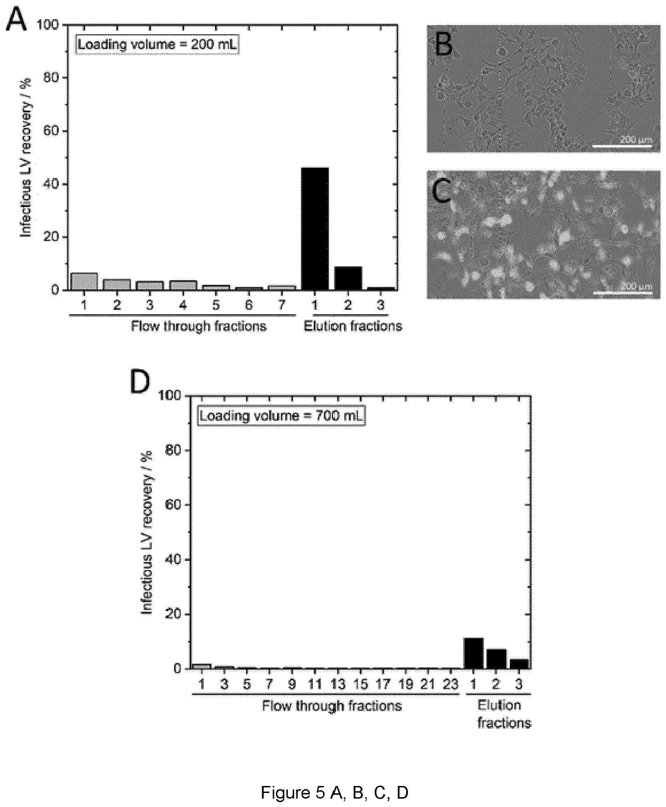

Next, different loading volumes ranging from 100 to 700 mL (corresponding to 50 to 350 mL LV solution) were tested on the MA100 device. The LV batch had a titer of 1.35×10⁷ TU·mL⁻¹ and 1.14×10¹⁰ VP·mL⁻¹. Previous SXC experiments with the MA100 were performed by loading 200 mL. Flow through and elution fractions of all runs were analyzed. No increase in the amount of LV in the flow through was observed as the loading volume increased (Fig. 5 A and D). These findings are supported by captured images of HEK293T cells expressing no GFP after transduction with the flow through fractions (Fig. 5 B). In contrast, HEK293T cells transduced with LV from the elution fractions showed GFP expression (Fig. 5 C). When a high LV amount was loaded (Fig. 5 D), the elution of the captured LV was hardly possible, resulting in a low recovery in the elution fraction.

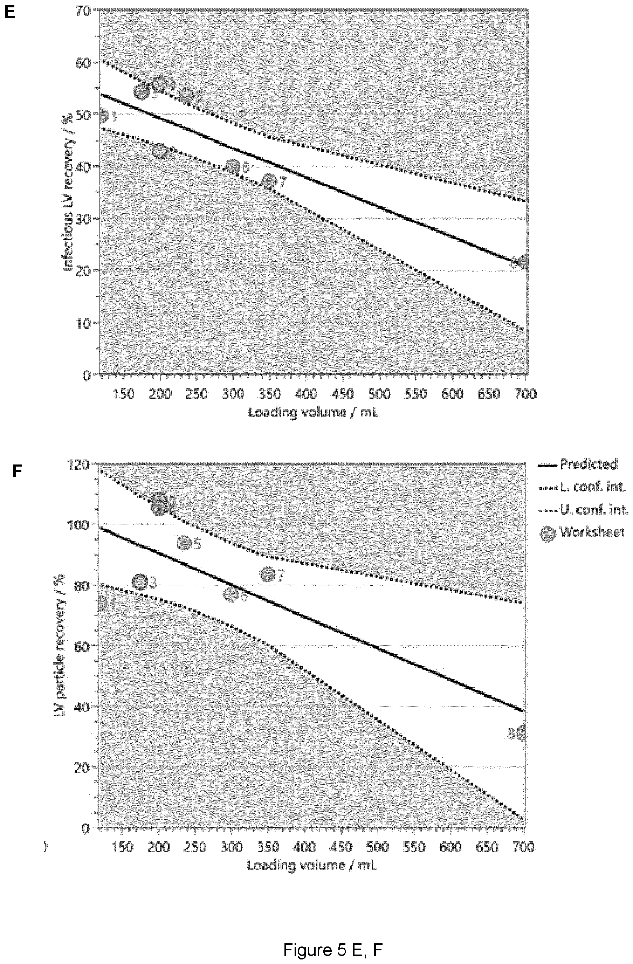

The highest infectious recovery of approximately 60% and particle recovery of about 100% was achieved loading around 200 mL (Fig. 5 E and F). Therefore, a loading capacity of 1.35 × 10⁹ TU and 1.14 × 10¹² VP was defined. In contrast to conventional chromatography methods, SXC does not rely on the stationary phase having functional groups (and limited binding sites as a result). As the visualization of LV on the membrane revealed, the LV is mainly captured on the upper membrane layers. Therefore, no LV breakthrough was observed. Hence, the membrane capacity for SXC cannot be defined at 10% LV breakthrough; instead, different loading volumes and the success of LV elution are analyzed to determine the loading capacity at which the LV recovery in the elution is satisfactory. In the previous experiments (Fig. 3 B), 4.10×10⁸ TU and 1.60×10⁹ TU were loaded onto the MA100 and MA15, respectively, showing that approximately 4× more LV could be loaded onto the MA100 device compared to the MA15 device. The loaded amount of LV was lower than in a previous study in which CAR-T-based LV was used with a higher LV titer in the loading material. These differences in the upstream material are likely the reason for the different outcomes, and it might be necessary to determine the ideal loading volume for each target product separately. Another reason could be the uneven LV distribution on the membrane with the MA100 standard housing discussed in Example 3, which might lead to an inefficient elution of overloaded membrane areas.

To further analyze the presented approach of scaling the flow rate according to the membrane surface area of the first layer, scale-down experiments with an axial PP15 device were performed for three different flow rates with N=3 each and a scale-up experiment with a radial 5" device for two different flow rates with N=1 each. Additionally, further runs at different flow rates with the MA15 and MA100 modules were performed with N=3 to complement the data.

According to literature, this is the first study using a membrane capsule for SXC and the largest membrane module used for this method to date with a loaded LV volume of 0.98 L. Pressure limitation was often discussed as a potential hurdle for SXC scale-up. As previously reported the viscous buffers result in a higher pressure compared to conventional chromatography methods like anion exchange chromatography, and a pressure increase during loading was often reported. A pressure increase was observed during the two scale-up runs with the 5" capsules from 0.4 to 0.8 bar (run 1) and 0.5 to 0.7 bar (run 2). As the pressure limit of the device is 4 bar, the pressure was no limiting factor during the scale-up runs under the tested conditions.

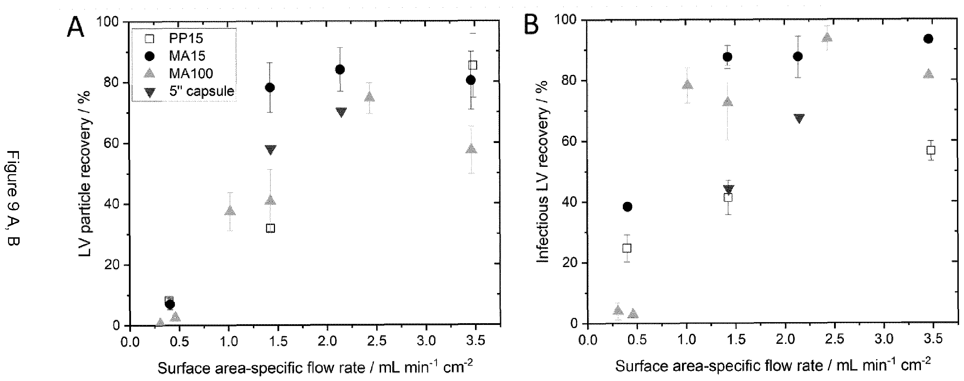

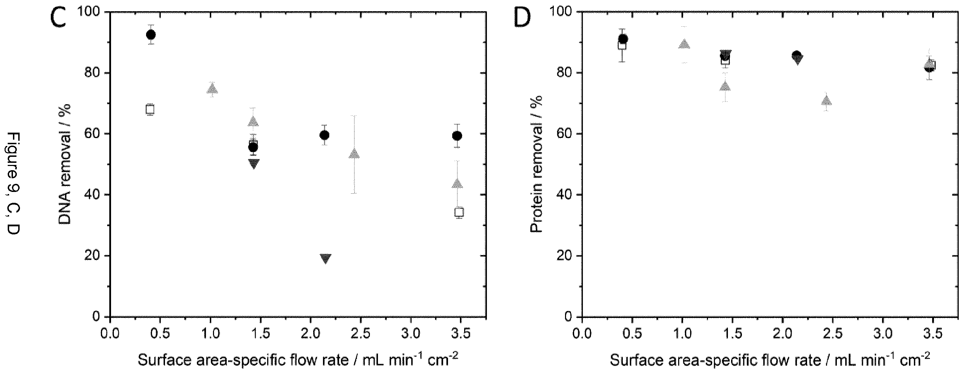

The infectious and particle recoveries, as well as impurity removals for the four different device scales are shown in Figure 9 plotted against different surface area-specific flow rates.

Plotting the infectious and particle titer recoveries of the tested device scales against different surface-area specific flow rates shows that if the flow rate falls under a critical minimum flow rate, the LV recovery decreases significantly (Fig. 9 A, B). It appears that the LV recovery approaches asymptotically a maximum. A decrease in LV recovery at flow rates above 3.5 mL·min⁻¹·cm⁻² is possible, however, there is a technically feasible limit due to the maximum flow rate of the system and the maximum pressure of the module. Further investigation is necessary to confirm this observation, but clearly, a surface area-dependent flow rate that is too low significantly reduces the LV recovery, and in general a surface area-dependent flow rate of approximately 1.4 mL·min⁻¹·cm⁻² and higher is necessary for a successful scale-up of SXC. When scaling the flow rate and loaded LV volume according to the membrane area of the chromatography module, the processing time for a complete chromatography run remains constant, thus SXC runs with an MA15 or a 5" capsule, both taking approximately 20 min at 1.43 mL·min⁻¹·cm⁻². This short processing time is especially beneficial for fragile enveloped viruses and viral vectors and enables a fast and efficient DSP process. With the scaling approach of using a minimum surface-area dependent flow rate, we were able to achieve a reproducible SXC LV recovery for four different module sizes. The highest LV volume purified by SXC was 980 mL with a recovery of 68% and represents an overall scaling factor of 98 compared with the smallest device PP15 (Table 1). The dsDNA removal shows a decreasing trend with increasing surface area-specific flow rate (Fig. 9 C). To achieve both, a high LV recovery and dsDNA removal a surface area-specific flow rate between 1.4 and 2.5 mL·min⁻¹·cm⁻² is preferred, reaching a dsDNA removal of approximately 51%. The protein removal was unaffected by the flow rate and consistent for the different module size with a protein removal of about 84% (Fig. 9 D). Overall good impurity removals were achieved and a subsequent ultrafiltration and diafiltration step will likely follow the DSP process to remove residual PEG and further increase the purity of the product.

### Example 3:

### Influence of the membrane housing design on SXC performance

After identifying critical process parameters for the scale-up of SXC with an axial chromatography device, the impact of the membrane housing design on LV capture on the membrane and SXC performance was investigated.

The membrane chromatography devices used in this study are operated by an axial flow from above through a membrane stack and have a low bed height (height of superimposed membrane layers). Besides a lower bed height, the incident flow area is larger than resin columns. A uniform flow distribution over the entire membrane area is required to avoid channeling and use the whole membrane area efficiently. An even flow distribution is achieved by a distributor structure inside the lid to spread the fluid over the membrane and a collector structure inside the table to collect the fluid. As the housing geometry significantly influences the fluid transport through the membrane, the housing design should play a major role during chromatography process development.

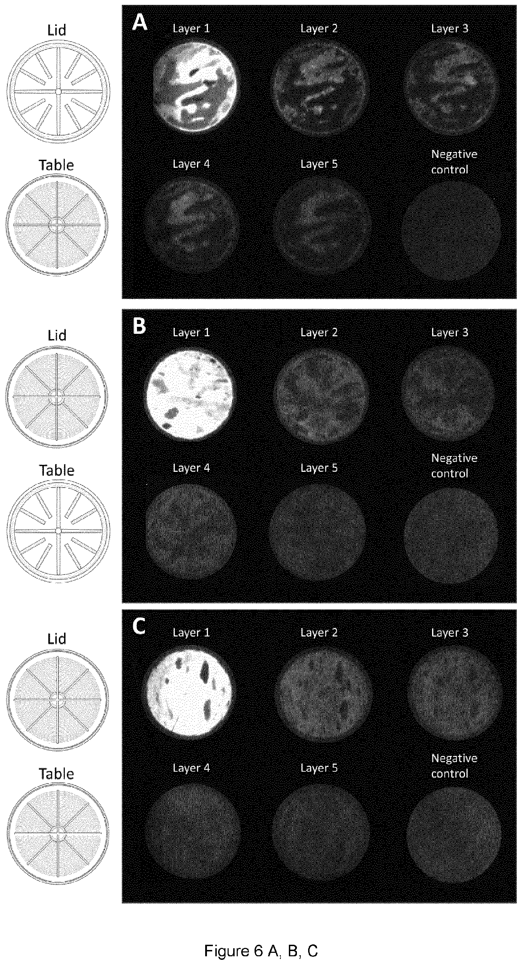

Lentiviral vector visualization with the MA100 housing was carried out to assess LV distribution on the membrane before elution. SXC was performed at a flow rate of 1.426 mL·min⁻¹·cm⁻². A volume of 160 mL was loaded, which corresponds to a volume of 80 mL LV solution with a particle titer of 7.27×10⁹ VP·mL⁻¹ and an infectious titer of 2.71×10⁷ TU·mL⁻¹. For the first SXC run with labeled LV, the standard housing configuration was used with the lid and table having distinct structures (Fig. 1 G and 1 H). Fig. 6 A displays an uneven distribution on the membrane layers with a consistent appearance throughout all membrane layers. As previously observed with the MA15 device (Fig. 2), the LV is mainly located on the upper layers, although some LV also reach the bottom membrane layers. The fluid does not seem to have been distributed evenly over the membrane. This uneven fluid distribution has favored membrane channeling and an imbalanced utilization of the membrane layer leading to the overloading of some areas.

For the second SXC run, the device's configuration was reversed; the table (Fig. 1 H) was used as a lid and vice versa. Therefore, the incoming fluid was distributed by the radial and circular distribution channels. Fig. 6 B shows that the LV is more evenly distributed on the membrane layers. LV presence on the first membrane layer is visible, comparable with the findings using the MA15 (Fig. 2). The changed lid and table configuration in this run highly improved the fluid distribution over the membrane. These findings demonstrate that a lid with radial and circular distribution channels is better suited to spread the fluid over the membrane than a coarse structure with thick bridges (Fig. 1 G). The dark spots within the bright areas indicate the presence of air bubbles that did not allow the fluid to access the membrane in this area. Air bubbles prevent the utilization of the surface area they occupy, reducing the recovery of target species. A higher pressure drop across the membrane could eliminate air bubbles.

A prototype was constructed with radial and circular distribution channels in the lid and the table. This housing configuration also resulted in evenly distributed LV on the membrane (Fig. 6 C). Some air bubbles were present in the device (dark spots). Comparing Fig. 6 B and 6 C, the LV appears to be better distributed with the prototype housing. A possible reason is that the fluid is not only evenly distributed on top of the membrane stack but is also collected from the membrane more efficiently and directed to the outlet of the table with the distribution channel design. This LV visualization experiment shows that the membrane module design is crucial to achieving evenly distributed fluid on the membrane so that the whole membrane area can be utilized.

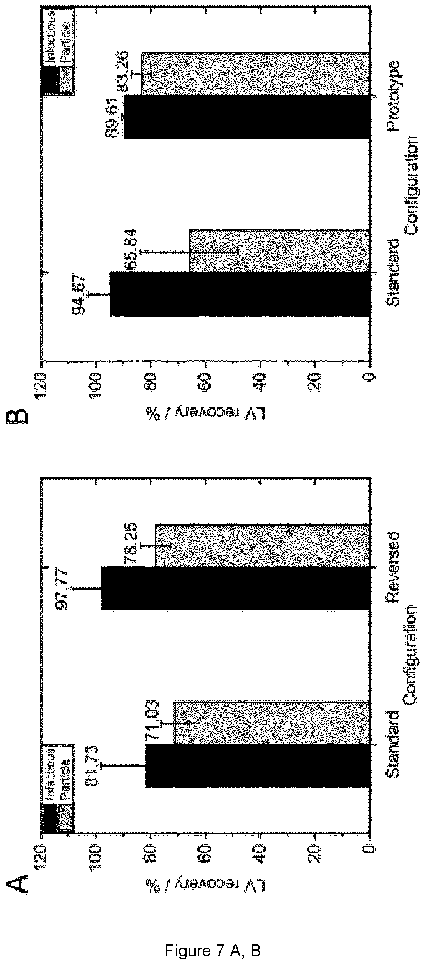

The three device configurations explained above were used to purify LV by SXC. For this experiment, the membranes, lid, and table of the MA100 were incorporated into a stainless-steel holder (Fig. 1 E). Hence, comparability to the overmolded MA100 devices used for previous experiments is limited. The LV recoveries for different MA100 housing configurations are shown in Fig. 7. The standard configuration was used for all previous experiments and served as a comparison with the reversed and prototype configuration. The virus solution purified with the standard and reversed configuration (Fig. 7 A) had a total particle titer of 1.02×10¹⁰ VP·mL⁻¹ and an infectious titer of 2.39×10⁷ TU·mL⁻¹. The virus solution purified with the prototype device and the standard configuration (Fig. 7 B) had a total particle titer of 4.32×10⁹ VP·mL⁻¹ with a concentration of 3.03×10⁷ TU·mL⁻¹.

The reversed configuration generated higher infectious LV recovery and total LV particle recovery compared with the standard configuration, although differences were not significant. LV recoveries of the prototype configuration were also not significantly different from the standard configuration, but standard deviations were lower with the prototype configuration. These findings indicate that utilizing a distribution structure on the inlet and outlet side allowed for generally more stable reproduction of LV recoveries. The prototype and reversed configuration reduce the dead volume on the inlet side, which decreases back mixing effects and promotes a narrower residence time distribution. Concerning the high LV recoveries, the uniform LV distribution on the membrane, and the lowest dead volume, the prototype device with the flow distributor plate in the lid and table is the favored configuration for an axial MA100 device.

### Conclusion:

Steric exclusion chromatography has been demonstrated to have the potential as a gentle purification method for large enveloped viral vectors. However, scale-up has not yet been investigated, raising the question of how to approach this challenge. Visualization of the LV on the membrane showed that SXC is a surface-oriented process, meaning that LV are mainly captured on the upper membrane layer. The present invention demonstrated that the flow rate must be scaled with the membrane area of the first layer. Scale-down and scale-up experiments demonstrate that a certain critical minimum surface area-dependent flow rate is necessary to achieve reproducible LV recoveries for the four different device scales tested with an overall scaling factor of 98. For the largest scale runs a radial device geometry was successfully used to purify 980 mL LV, and further scale-up could be realized by using larger capsules or cassette modules. Investigating various loading volumes showed no LV breakthrough with increasing volume. However, the elution of LV from overloaded membrane areas was hardly possible, indicating an optimal amount of LV to be loaded. Altering the design of the MA100 module housing improved the flow distribution and led to a uniform distribution of LV on the membrane. The use of the improved housing prototypes could offer the possibility to load more LV, as overloading of membrane areas is more likely to be avoided. Overall, the scalability of SXC using membrane modules is demonstrated in the present invention, providing a basis for potential future industrial applications of the method.

## Claims

1. A method for the purification of viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, by filtration-supported polyalkylene glycol precipitation, comprising the steps of:
(a) providing a filtration module, said filtration module comprising:
(i-α) one stack or two separate stacks of 2 to 9 layers of a stabilized, non-ionic, hydrophilic porous structure, said layers being deposited on top of each other, wherein each of said stack(s) provide(s) a homogenous flow resistance and has a total thickness of 300 µm or more and 2000 µm or less, or
(i-β) one monolithic stabilized, non-ionic, hydrophilic porous structure, or two separate monolithic stabilized, non-ionic, hydrophilic porous structures, wherein each of said porous structure(s) provide(s) a homogenous flow resistance and has a total thickness of 300 µm or more and 2000 µm or less, and
(ii) a housing encasing said stack(s) of porous structure layers or said monolithic porous structure(s), said housing comprising a fluid inlet and a fluid outlet, wherein fluid entering the filtration module through the fluid inlet has to pass through said stack(s) of porous structure layers or said monolithic porous structure(s) in order to exit the filtration module through the fluid outlet, and wherein the housing is adapted to distribute the feed solution entering the housing via the fluid inlet evenly over the entire accessible surface area of the first layer of said stack(s) of porous structure layers or over the entire accessible surface area of said monolithic porous structure(s),
(b) providing a feed solution, containing said viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, to said filtration module via said fluid inlet at a surface area-dependent flow rate of at least 1.0 mL per minute per cm² of accessible surface area of the first layer of said stack(s) of layers of a porous structure or of the monolithic porous structure(s) (1.0 mL·min⁻¹·cm⁻²), whereby the viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, adhere to the porous structure, and
(c) eluting the viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more, from said porous structure and thereby harvesting purified viruses, virus-like particles, extracellular vesicles, proteins having a diameter of 5 nm or more, protein complexes having a diameter of 5 nm or more, nucleic acids having a diameter of 5 nm or more, protein-nucleic acid complexes having a diameter of 5 nm or more, and/or molecules having a diameter of 5 nm or more.

2. The method of claim 1, wherein the stack of porous structure layers is a stack of 3 to 5 porous structure layers.

3. The method of claim 1 or claim 2, wherein said porous structures have a pore size of 1 to 5 µm.

4. The method of any one of claims 1 to 3 wherein each of said porous structure layers has a thickness of 100 to 300 µm.

5. The method of any one of claims 1 to 4, wherein the uppermost layer of said stack of porous structure layers has an accessible diameter of more than 25 mm, and an accessible surface area of more than 4.91 cm².

6. The method of any one of claims 1 to 5, wherein the porous structure is a stabilized cellulose membrane.

7. The method of any one of claims 1 to 6, wherein the filtration module is an axial flow module, a capsule module, or a cassette module.

8. The method of any one of claims 1 to 7, wherein prior to step (b), the feed solution is mixed with a buffer in a ratio of buffer: feed solution of 4:1 to 1:4.

9. The method of claim 8, wherein said buffer contains a polyalkylene glycol having two or three carbon atoms in its repeating unit.

10. The method of claim 9, wherein the polyalkylene glycol is polyethylene glycol (PEG).

11. The method of claim 10, wherein the PEG is a PEG having a molar mass of between 400 and 12000 g/mol.

12. The method of claim 10 or claim 11, wherein the buffer contains the polyalkylene glycol or PEG in an amount of 4 to 20 wt.-% final polyalkylene glycol or PEG concentration after mixing with the feed solution.

13. The method of any one of claims 1 to 12, wherein elution in step (c) is conducted in an upflow flow direction.

14. The method of any one of claims 1 to 13, wherein the virus or the respective virus-like particle is selected from the group consisting of lentiviruses, baculoviruses, Orf virus, adeno-associated viruses (AAV), influenza viruses (such as influenza A virus), yellow fever viruses, human papillomaviruses, vaccinia viruses, adenoviruses, hepatitis viruses, polioviruses, rabies viruses, rotaviruses, rubellaviruses, Zika viruses, and respective virus-like particles.

15. A filtration module, comprising:
(a-α) one stack or two separate stacks of 2 to 9 layers of a stabilized, non-ionic, hydrophilic porous structure, said layers being deposited on top of each other, wherein each of said stack(s) provide(s) a homogenous flow resistance and has a total thickness of 300 µm or more and 2000 µm or less, or
(a-β) one monolithic stabilized, non-ionic, hydrophilic porous structure, or two separate monolithic stabilized, non-ionic, hydrophilic porous structures, wherein each of said porous structure(s) provide(s) a homogenous flow resistance and has a total thickness of 300 µm or more and 2000 µm or less, and
(b) a housing encasing said stack(s) of porous structure layers, said housing comprising a fluid inlet and a fluid outlet, wherein fluid entering the filtration module through the fluid inlet has to pass through said stack(s) of porous structure layers in order to exit the filtration module through the fluid outlet, and wherein the housing is adapted to distribute the feed solution entering the housing via the fluid inlet evenly over the entire accessible surface area of the first layer of said stack(s) of porous structure layers.
